# EUROPEAN PATENT SPECIFICATION

(11) **EP 4 329 621 B1**
(45) Date of publication and mention of the grant of the patent: **27.08.2025**
(21) Application number: 23759341.3
(22) Date of filing: 28.02.2023
(51) Int. Cl.: A61B 6/03, A61B 6/00, G06T 11/00, G06T 19/20

(54) **METHODS, SYSTEMS, AND STORAGE MEDIUMS FOR IMAGE GENERATION**
VERFAHREN, SYSTEME UND SPEICHERMEDIEN ZUR BILDERZEUGUNG
PROCÉDÉS, SYSTÈMES ET SUPPORTS DE STOCKAGE POUR LA GÉNÉRATION D'IMAGES

(30) Priority: 28.02.2022 CN 202210189999
(43) Date of publication of application: 06.03.2024
(73) Proprietor: Shanghai United Imaging Healthcare Co., Ltd., Shanghai 201807 (CN)
(72) Inventor: LIU, Yanyan, Shanghai 201807 (CN)
(74) Representative: Wang, Bo
(86) International application number: PCT/CN2023/078736
(87) International publication number: WO 2023/160720

(56) References cited:
- EP-A1- 3 560 424
- CN-A- 111 915 696
- CN-A- 114 533 103
- JP-A- 2005 270 652
- JP-A- 2008 532 612
- US-A1- 2003 220 555
- US-A1- 2011 282 189
- TANZI LEONARDO ET AL: "Real-time deep learning semantic segmentation during intra-operative surgery for 3D augmented reality assistance", INTERNATIONAL JOURNAL OF COMPUTER ASSISTED RADIOLOGY AND SURGERY, SPRINGER, DE, vol. 16, no. 9, 24 June 2021 (2021-06-24), pages 1435 - 1445, XP037534958, ISSN: 1861-6410, [retrieved on 20210624], DOI: 10.1007/S11548-021-02432-Y
- UNBERATH MATHIAS ET AL: "The Impact of Machine Learning on 2D/3D Registration for Image-Guided Interventions: A Systematic Review and Perspective", FRONTIERS IN ROBOTICS AND AI, vol. 8, 30 August 2021 (2021-08-30), XP093198587, ISSN: 2296-9144, DOI: 10.3389/frobt.2021.716007

## Description

### CROSS-REFERENCE TO RELATED APPLICATIONS

This application claims priority to Chinese Patent Application No. 202210189999.3, filed on February 28, 2022.

### TECHNICAL FIELD

The present disclosure relates to the field of medical imaging, and in particular, to systems, methods, and storage mediums for image generation.

### BACKGROUND

Computed tomography (CT) is one of the most general imaging technologies in the medical field, which can be used to obtain a three-dimension (3D) image of a scanned subject. In some cases, a same target subject needs to be imaged multiple times to generate a plurality of 3D images of the target subject. For example, before intervention therapy, an image which only contains tissues may be obtained by a CT scan when an interventional needle has not entered the target subject; after the interventional needle enters the internal of the target subject, a plurality of reconstructed images need to be obtained by multiple CT scans to determine a real-time position of the interventional needle and guide doctors to place the interventional needle in a specific position. As another example, changes of lesion location over time may be examined by performing multiple CT scans on the target subject. However, repeated CT scans cause doctors and patients to be exposed to a radiation field for a long time, which may damage the health of the doctors and patients. Moreover, a 3D image reconstructed based on the 3D CT scan usually exists hardening artifacts of the interventional needle, which makes it difficult to judge a tip of the interventional needle and affects the accuracy of the intervention therapy.

Therefore, it is desirable to provide methods, systems, and storage mediums for image generation to eliminate the tip artifacts and reduce scanning doses.

Patent Publication CN 111915696 A constitutes relevant prior art as it addresses the issue of high radiation doses from repeated CT scans. It proposes a low-dose scan data reconstruction method utilizing existing 3D image data of the same patient. By analyzing the difference between the prior high-dose data and the new low-dose scan (CT or DR), the method reconstructs a high-quality 3D image suitable for diagnosis. This approach reduces the need for high radiation doses, leverages existing patient information, and can improve image quality by addressing artifacts from metal implants or contrast agents.

### SUMMARY

The invention is captured and limited by the appended claims.

One aspect of the present disclosure may provide a method for image generation implemented on a computing device having one or more processors and one or more storage devices. The method may comprise: obtaining a three-dimensional (3D) base image of a target subject, the 3D base image being captured by performing a 3D scan on the target subject with a preset posture; obtaining one or more two-dimensional (2D) scout images of the target subject, the one or more 2D scout images of the target subject being captured by performing a scout scan on the target subject with the preset posture after an internal structure of the target subject changes; generating a 3D predicted image of the subject based on the 3D base image and the one or more 2D scout images, the 3D predicted image indicating the internal structure of the target subject when the one or more 2D scout images are captured.

In some embodiments, the generating a 3D predicted image of the subject may comprise: generating the 3D predicted image by processing the 3D base image and the one or more 2D scout images using an image generation model, wherein the image generation model may be a machine learning model.

In some embodiments, the internal structure of the target subject may change due to at least one of an intervention of an intervention equipment into the target subject or physiological change of a region of interest of the target subject.

In some embodiments, the method may further comprise: obtaining reference information relating to at least one of an intervention point of the intervention equipment, a moving route of the intervention equipment, a shape of the intervention equipment, a material of the intervention equipment, or a type of the intervention equipment, wherein the 3D predicted image may be generated further based on the reference information.

In some embodiments, the image generation model may be trained using a plurality of training samples, each of which may include: a ground truth 3D image and one or more sample 2D scout images indicating the internal structure of a sample subject with a sample intervention equipment inside the sample subject, and a sample 3D base image indicating the internal structure of the sample subject without the sample intervention equipment inside the sample subject.

In some embodiments, a training sample of the plurality of training samples may be generated by: generating the sample 3D base image by performing a first 3D scan on the sample subject before the sample intervention equipment is inserted into the sample subject; generating the ground truth 3D image by performing a second 3D scan on the sample subject after the sample intervention equipment is inserted into the sample subject; and generating the one or more sample 2D scout images based on the ground truth 3D image.

In some embodiments, a training sample of the plurality of training samples may be generated by: generating the ground truth 3D image by performing a third 3D scan on the sample subject after the sample intervention equipment is inserted into the sample subject; and generating the sample 3D base image and the one or more sample 2D scout images based on the ground truth 3D image.

In some embodiments, a training sample of the plurality of training samples may be generated by: obtaining a digital model representing the sample subject; generating the sample 3D base image by simulating a 3D scan on the digital model; and generating the ground truth 3D image and the one or more sample 2D scout images based on the sample 3D base image.

In some embodiments, the 3D base image may include a plurality of frames corresponding to a plurality of physiological phases of the target subject, and the generating a 3D predicted image of the subject by processing the 3D base image and the one or more 2D scout images using the image generation model may comprise: determining a target physiological phase of the target subject corresponding to the one or more 2D scout images; selecting, from the plurality of frames, a target frame corresponding to the target physiological phase; and generating the 3D predicted image by processing the target frame and the one or more 2D scout images using the image generation model.

In some embodiments, the generating the 3D predicted image by processing the 3D base image and the one or more 2D scout images using the image generation model may comprise: generating a preliminary 3D predicted image by processing the 3D base image and the one or more 2D scout images using the image generation model; and generating the 3D predicted image by performing artifact correction on the preliminary 3D predicted image.

In some embodiments, the artifact correction may be performed using an artifact correction model, which is jointly trained with the image generation model.

Another aspect of the present disclosure may provide a system for image generation, implemented on a computing device having one or more processors and one or more storage devices, the system comprising: at least one storage device including a set of instructions; and at least one processor configured to communicate with the at least one storage device, wherein when executing the set of instructions, the at least one processors may be configured to direct the system to perform operations including: obtaining a three-dimensional (3D) base image of a target subject, the 3D base image being captured by performing a 3D scan on the target subject with a preset posture; obtaining one or more two-dimensional (2D) scout images of the target subject, the one or more 2D scout images of the target subject being captured by performing a scout scan on the target subject with the preset posture after an internal structure of the target subject changes; generating a 3D predicted image of the subject based on the 3D base image and the one or more 2D scout images, the 3D predicted image indicating the internal structure of the target subject when the one or more 2D scout images are captured.

In some embodiments, the generating a 3D predicted image of the subject may comprise: generating the 3D predicted image by processing the 3D base image and the one or more 2D scout images using an image generation model, wherein the image generation model may be a machine learning model.

In some embodiments, the internal structure of the target subject may change due to at least one of an intervention of an intervention equipment into the target subject or physiological change of a region of interest of the target subject.

In some embodiments, the method may further comprise: obtaining reference information relating to at least one of an intervention point of the intervention equipment, a moving route of the intervention equipment, a shape of the intervention equipment, a material of the intervention equipment, or a type of the intervention equipment, wherein the 3D predicted image may be generated further based on the reference information.

In some embodiments, the image generation model may be trained using a plurality of training samples, each of which may include: a ground truth 3D image and one or more sample 2D scout images indicating the internal structure of a sample subject with a sample intervention equipment inside the sample subject, and a sample 3D base image indicating the internal structure of the sample subject without the sample intervention equipment inside the sample subject.

In some embodiments, a training sample of the plurality of training samples may be generated by: generating the sample 3D base image by performing a first 3D scan on the sample subject before the sample intervention equipment is inserted into the sample subject; generating the ground truth 3D image by performing a second 3D scan on the sample subject after the sample intervention equipment is inserted into the sample subject; and generating the one or more sample 2D scout images based on the ground truth 3D image.

In some embodiments, a training sample of the plurality of training samples may be generated by: generating the ground truth 3D image by performing a third 3D scan on the sample subject after the sample intervention equipment is inserted into the sample subject; and generating the sample 3D base image and the one or more sample 2D scout images based on the ground truth 3D image.

In some embodiments, a training sample of the plurality of training samples may be generated by: obtaining a digital model representing the sample subject; generating the sample 3D base image by simulating a 3D scan on the digital model; and generating the ground truth 3D image and the one or more sample 2D scout images based on the sample 3D base image.

Another aspect of the present disclosure may provide a non-transitory computer readable medium, comprising at least one set of instructions, wherein when executed by one or more processors of a computing device, the at least one set of instructions may cause the computing device to perform a method, the method comprising: obtaining a three-dimensional (3D) base image of a target subject, the 3D base image being captured by performing a 3D scan on the target subject with a preset posture; obtaining one or more two-dimensional (2D) scout images of the target subject, the one or more 2D scout images of the target subject being captured by performing a scout scan on the target subject with the preset posture after an internal structure of the target subject changes; generating a 3D predicted image of the subject based on the 3D base image and the one or more 2D scout images, the 3D predicted image indicating the internal structure of the target subject when the one or more 2D scout images are captured.

Additional features will be set forth in part in the description which follows, and in part will become apparent to those skilled in the art upon examination of the following and the accompanying drawings or may be learned by production or operation of the examples. The features of the present disclosure may be realized and attained by practice or use of various aspects of the methodologies, instrumentalities, and combinations set forth in the detailed examples discussed below.

### BRIEF DESCRIPTION OF THE DRAWINGS

The present disclosure is further illustrated in terms of exemplary embodiments, and these exemplary embodiments are described in detail with reference to the drawings. These embodiments are not restrictive. In these embodiments, the same number indicates the same structure, wherein:
FIG. 1 is a schematic diagram illustrating an exemplary image generation system according to some embodiments of the present disclosure;
FIG. 2 is a block diagram illustrating an exemplary processing device according to some embodiments of the present disclosure;
FIG. 3 is a flowchart illustrating an exemplary process for image generation according to some embodiments of the present disclosure;
FIG. 4 is a flowchart illustrating an exemplary process for generating a training sample of an image generation model according to some embodiments of the present disclosure;
FIG. 5 is a flowchart illustrating an exemplary process for generating a training sample of an image generation model according to some embodiments of the present disclosure;
FIG. 6 is a flowchart illustrating an exemplary process for generating a training sample of an image generation model according to some embodiments of the present disclosure;
FIG. 7 is a schematic diagram illustrating an exemplary method for training an image generation model according to some embodiments of the present disclosure; and
FIG. 8 is a schematic diagram illustrating another exemplary method for training an image generation model according to some embodiments of the present disclosure.

### DETAILED DESCRIPTION

In the following detailed description, numerous specific details are set forth by way of examples in order to provide a thorough understanding of the relevant disclosure. However, it should be apparent to those skilled in the art that the present disclosure may be practiced without such details. In other instances, well-known methods, procedures, systems, components, and/or circuitry have been described at a relatively high-level, without detail, in order to avoid unnecessarily obscuring aspects of the present disclosure. Various modifications to the disclosed embodiments will be readily apparent to those skilled in the art, and the general principles defined herein may be applied to other embodiments and applications without departing from the scope of the present disclosure. Thus, the present disclosure is not limited to the embodiments shown, but to be accorded the widest scope consistent with the claims.

It will be understood that the term "system," "engine," "unit," "module," and/or "block" used herein are one method to distinguish different components, elements, parts, section or assembly of different level in ascending order. However, the terms may be displaced by another expression if they achieve the same purpose.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including," when used in this specification, specify the presence of stated features, integers, steps, operations, elements, and/or components, but do not preclude the presence or addition of one or more other features, integers, steps, operations, elements, components, and/or groups thereof.

flowcharts are used in the present disclosure to illustrate the operation performed by the system according to the embodiment of the present disclosure. It should be understood that the preceding or subsequent operations are not necessarily performed accurately in sequence. Instead, the steps may be processed in reverse order or simultaneously. At the same time, other operations may add to these procedures, or remove one or more operations from these procedures.

The term "image" in the present disclosure may refer to image data (e.g., scanning data, projection data) and/or images in various forms, including two-dimensional (2D) images, three-dimensional (3D) images, etc. The term "pixel" and "voxel" in the present disclosure may be used interchangeably to refer to elements of an image. In the present disclosure, a representation of a subject (e.g., an object, a patient, or a portion thereof) in an image may be referred to as "subject" for brevity. For instance, a representation of an organ, tissue (e.g., a heart, a liver, a lung), or a region of interest (ROI) in an image may be referred to as the organ, tissue, or ROI, for brevity. Further, an image including a representation of a subject may be referred to as an image of the subject or an image including the subject for brevity. Still further, an operation performed on a representation of a subject in an image may be referred to as an operation performed on the subject for brevity. For instance, a segmentation of a portion of an image including a representation of an ROI from the image may be referred to as a segmentation of the ROI for brevity.

In some embodiments of the present disclosure, a real-time three-dimension (3D) predicted image may be generated by a deep learning model based on a base 3D image and one or more two-dimensional (2D) scout images, which improves an imaging speed. In some embodiments, ground truth images used for model training may be generated by data simulation manners such as using a digital model, etc., or by correcting a real image, which can greatly reduce and eliminate tip artifacts in the real-time 3D image and improve the image quality. By generating the real-time 3D predicted image based on the 2D scout image(s) instead of performing a 3D scan, the time that doctors and patients are exposed to a radiation field can be reduced, which can greatly reduce scanning doses received by the doctors and patients, and protect the health of the doctors and patients.

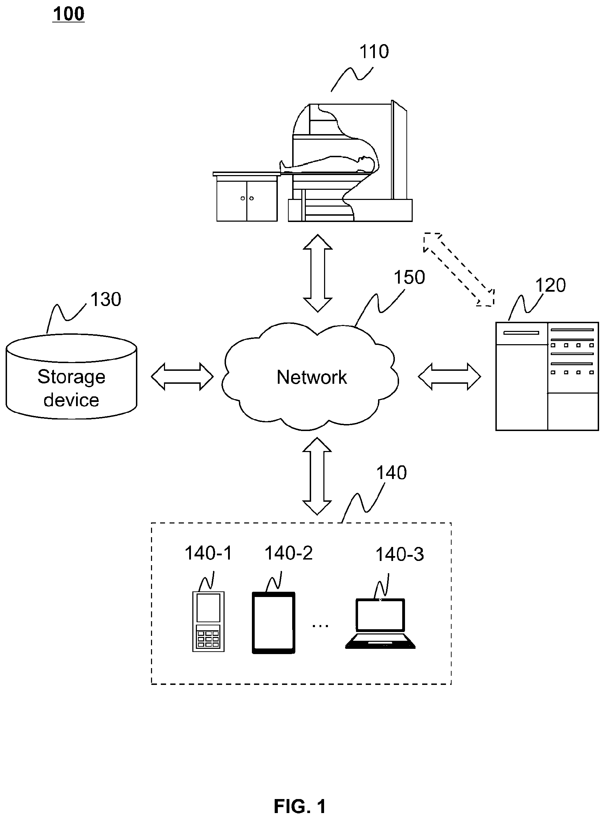

FIG. 1 is a schematic diagram illustrating an exemplary image generation system 100 according to some embodiments of the present disclosure. For brevity, the imaging generation system 100 is also referred to as the system 100. As shown in FIG. 1, in some embodiments, the system 100 may include a medical imaging device 110, a processing device 120, a storage device 130, a terminal 140, and a network 150.

The medical imaging device 110 may refer to a medical device that generates an image reflecting an internal structure of a human body. In some embodiments, the medical imaging device 110 may include a single-modality scanning device and/or a multi-modality scanning device. The signal-modality scanning device may include scanning devices such as a computed tomography (CT) device, a magnetic resonance imaging (MRI) device, etc.; the multi-modality scanning device may include scanning devices such as a single-photon emission computed tomography-magnetic resonance imaging (SPECT-MRI) device, a positron emission tomography-computed tomography (PET-CT) device, etc.

In some embodiments, when the target subject (i.e., a scanned subject) is placed in a scanning position, a radiation source of the medical imaging device 110 may emit radioactive rays to the target subject, a detector 110 in the medical imaging device 110 may receive radiation emitted by the radiation source and measure the received radiation. In some embodiments, the medical imaging device 110 may transmit data captured in a scan of the target subject to the processing device 120, wherein the data may include scanning images (e.g., the 2D image, the 3D image, etc.) of the target subject and/or original scanning data used to generate the scanning images. The medical imaging device 110 may receive instructions input by a user through the terminal 140 and perform relevant operations according to the instructions, such as imaging, etc. In some embodiments, the medical imaging device 110 may communicate data with other components of the system 100 (e.g., the processing device 120, the storage device 130, the terminal 140) via the network 150. In some embodiments, the medical imaging device 110 may be connected with other components of the system 100 directly. In some embodiments, one or more components of the system 100 (e.g., the processing device 120, the storage device 130) may be integrated into the medical imaging device 110.

The processing device 120 may process data obtained from components of the system 100 to perform image generation methods disclosed in the present disclosure. For example, the processing device 120 may generate a 3D predicted image of the target subject based on a 3D base image and one or more 2D scout images of the target subject. As another example, the processing device 120 may generate an image generation model through model training. In some embodiments, the processing device 120 may obtain pre-store data from the storage device 130, such as scanning images of the target subject, an initial image generation model, a trained image generation model, training samples used for the model training, etc. In some embodiments, the processing device 120 may perform the image generation methods illustrated in some embodiments of the present disclosure, for example, a method for generating the 3D predicted image of the target subject using the trained image generation model, etc.

In some embodiments, the processing device 120 may include one or more sub-processing devices (e.g., single-core processing devices or multi-core processing devices). Merely for example, the processing device 120 may include a central processing unit (CPU), an application specific integrated circuit (ASIC), an application special instruction processor (ASIP), a graphics processing unit (GPU), a physics processing unit (PPU), a digital signal processor (DSP), a field programmable gate array (FPGA), a programmable logic device (PLD), a controller, a microcontroller unit, a reduced instruction set computer (RISC), a microprocessor, etc., or any combination thereof.

The storage device 130 may store data or information generated by other devices. In some embodiments, the storage device 130 may store data and/or information being captured by the medical image device 110, such as the 3D base image, etc. In some embodiments, the storage device 130 may store data and/or information processed by the processing device 120, such as the trained image generation model, the training sample used for the model training, the 3D predicted image of the target subject, etc. The storage device 130 may include one or more storage components, each storage component may be an independent device or a part of other devices. The storage device may be local or cloud based.

The terminal 140 may implement interaction between users and one or more components of the system 100. A user may input operation instructions for controlling the medical imaging device 110 via the terminal 140, which enables the medical imaging device 110 to complete specific operations, such as scanning specific body parts of a patient. In some embodiments, the terminal 140 may instruct the processing device 120 to perform the image generation methods illustrated in some embodiments of the present disclosure. In some embodiments, the terminal 140 may receive the 3D predicted image of the target subject from the processing device 120, a user may determine a current position of an intervention equipment (e.g., an interventional needle) in the interventional therapy according to the 3D predicted image so as to perform the interventional therapy on the target subject accurately and effectively. In some embodiments, the user may determine whether the target subject changes over time according to the received 3D predicted image to make targeted diagnosis and treatment. In some embodiments, the terminal 140 may be one or more combinations of devices with input and/or output functions. Exemplary terminal 140 may include a mobile device 140-1, a tablet computer 140-2, a laptop computer 140-3, a desktop computer, etc.

The network 150 may connect various components of the system and/or connect the system with external resources. The network 150 may enable communications between various components of the system 100 or other components outside the system 100 to facilitate the exchange of data. In some embodiments, one or more components in the system 100 (e.g., the medical imaging device 110, the processing device 120, the storage device 130, the terminal 140) may emit data to other components via the network 150. In some embodiments, the network 150 may include wired networks and/or wireless networks.

It should be noted that the above descriptions are provided for illustrative purposes only and not intended to limit the scope of the present disclosure. For those skilled in the art, many changes and modifications can be made under the guidance of the content of the present disclosure. The characteristic, structure, method, and other features of the exemplary embodiments described in the present disclosure can be combined in various manners to obtain other and/or alternative exemplary embodiments. For example, the processing device 120 may be based on a cloud computing platform, such as a public cloud, a private cloud, a community, a hybrid cloud, or the like. However, these changes and modifications may not deviate from the scope of the present disclosure.

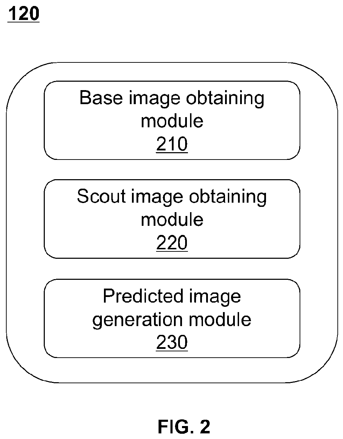

FIG. 2 is a block diagram illustrating an exemplary processing device according to some embodiments of the present disclosure. As shown in FIG. 2, in some embodiments, the processing device 120 may include a base image generation obtaining module 210, a scout image obtaining module 220, and a predicted image generation module 230.

In some embodiments, the base image obtaining module 210 may be configured to obtain a three-dimension (3D) base image of a target subject. The 3D base image may be captured by performing a 3D scan on the target subject with a preset posture. More descriptions regarding the 3D base image may be found elsewhere in the present disclosure. See, e.g., operation 310 and relevant descriptions thereof.

In some embodiments, the scout image obtaining module 220 may be configured to obtain one or more two-dimensional (2D) scout images of the target subject. The one or more 2D scout images of the target subject may be captured by performing a scout scan on the target subject with the preset posture after an internal structure of the target subject changes. More descriptions regarding the 2D scout image(s) may be found elsewhere in the present disclosure. See, e.g., operation 320 and relevant descriptions thereof.

In some embodiments, the predicted image generation module 230 may be configured to a 3D predicted image of the subject based on the 3D base image and the one or more 2D scout images. The 3D predicted image may indicate the internal structure of the target subject when the one or more 2D scout images are captured. More descriptions regarding the generation of the 3D predicted image may be found elsewhere in the present disclosure. See, e.g., operation 330 and relevant descriptions thereof.

In some embodiments, the processing device 120 may include a reference information obtaining module (not shown in figures) configured to obtain reference information. The reference information may relate to at least one of an intervention point of an intervention equipment, a moving route of the intervention equipment, a shape of the intervention equipment, a material of the intervention equipment, or a type of the intervention equipment. In some embodiments, the 3D predicted image may be generated further based on the reference information.

In some embodiments, the processing device 120 may further include a model training module (not shown in figures). The model training module may be configured to train an image generation model and/or an artifact correction model.

It should be noted that the above description of the processing device 120 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. For example, the processing device 120 may include one or more additional modules, such as a storage module (not shown) for storing data. As another example, one or more modules of the processing device 120 described above may be omitted. Additionally or alternatively, two or more modules of the processing device 120 may be integrated into a single component. A module of the processing device 120 may be divided into two or more sub-modules.

In some embodiments, the model training module and other modules described above may be implemented on different computing devices. Merely by way of example, the model training module may be implemented on a computing device of a vendor of the machine learning model(s), while the other modules described above may be implemented on a computing device of a user of the machine learning model(s).

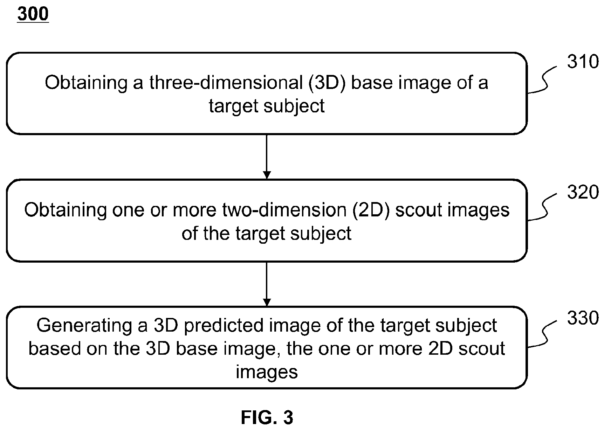

FIG. 3 is a flowchart illustrating an exemplary process for image generation according to some embodiments of the present disclosure.

In some embodiments, process 300 may be executed by a processing device (e.g., the processing device 120). For illustration purposes, the following descriptions are described with reference to image generation in CT, and not intended to limit the scope of the present disclosure. It should be noted that the process 300 can also be used to generate images of any other forms, such as MRI images, PET images, or the like, or any combination thereof.

In some embodiments, the process 300 may be used in an interventional therapy. In the interventional therapy, medical imaging (e.g., CT imaging) may be used as guidance tools to guide the intervention equipment to a specified position inside the target object. During the interventional therapy, if the position of the intervention equipment in the target subject changes, an internal structure of the target subject may change accordingly. In general, the position of the intervention equipment in the target subject may be tracked by performing 3D CT scans continuously. However, traditional 3D CT scans (e.g., spiral CT scans or axial CT scans) have greater scanning dose and longer scan duration compared with a scout CT scan. In particular, when real-time scanning images are required to track the change of the scanned body part continuously, doctors and patients may be exposed to a high-dose radiation field for a long time. The process 300 may be performed in the interventional therapy. Specifically, after performing a 3D CT imaging with a relatively larger dose and a relatively longer time, a CT position scout scan with a relatively smaller dose and a relatively shorter time may be performed and used to predict a real-time position of the intervention equipment, which can reduce risks for the doctors and patients. During the intervention process, the imaging speed may be required to be as fast as possible or even real-time. The process 300 may be used to perform the intervention imaging with a fast imaging speed, thereby achieving real-time imaging.

In some embodiments, the process 300 may be used in repeat examination. The repeat examination may refer to performing one or more repeated 3D scans on a region of interest (ROI) for tracking changes of the ROI after the first 3D CT imaging has been performed on the ROI. For example, the ROI may be lesion regions, such as regions with tumor, inflammation, fracture, that may change over time. During the repeat examination, the repeat scans may make the doctors and patients receive high radiation doses for a long time, which may affect their health. During the repeat examination, by using the process 300, 2D scout image(s) may be obtained by using a scout CT scan with relatively smaller doses and a relatively shorter time. Then, a current predicted 3D image may be obtained based on a base 3D image obtained by a first examination and scout image(s) obtained by a new scan, which can reduce the radiation doses received by the doctors and patients and reduce the risks of the doctors and patients.

As shown in FIG. 3, the process 300 may include the following operations.

In 310, a 3D base image of a target subject may be obtained. The 3D base image may be captured by performing a 3D scan on the target subject with a preset posture. In some embodiments, the operation 310 may be executed by the base image obtaining module 210 of the processing device 120.

The 3D base image may refer to a 3D scanning image used as a reference base, such as a 3D CT scanning image, etc. The target subject may refer to a subject that needs to be examined and/or treated, wherein an internal structure of the target subject may change during the examination and/or treatment. For example, the target subject may be a patient that needs to receive an interventional therapy or a repeat examination. In some embodiments, when the target subject is the patient that needs to receive the interventional therapy, the 3D base image may include a 3D scanning image acquired when an intervention equipment (e.g., an interventional needle) has not been inserted into the body of the target subject. In some embodiments, when the target subject is the patient that needs to receive the repeat examination, the 3D base image may include a 3D scanning image captured by one of examinations of the target subject.

In some embodiments, the processing device 120 may obtain the 3D base image by directing a medical imaging device (e.g., the medical imaging device 110) to perform a 3D scan on the target subject, wherein the target subject may hold the preset posture during the 3D scan. The preset posture may include a standard posture used for the treatment (e.g., the interventional therapy), examination (e.g., disease reexamine), or the like, such as a supine position, a lateral position, etc.

In some embodiments, the processing device 120 may obtain the 3D base image of the target subject by other manners. For example, the processing device 120 may obtain the 3D base image from a storage device (e.g., the storage device 130). As another example, the processing device 120 may generate the 3D base image by performing image reconstruction on scan data collected by the medical imaging device in the 3D scan.

In 320, one or more 2D scout images of the target subject may be obtained. The one or more 2D scout images of the target subject may be captured by performing a scout scan on the target subject with the preset posture after an internal structure of the target subject changes. In some embodiments, the operation 320 may be executed by the scout image obtaining module 220 of the processing device 120.

The count of the 2D scout image(s) may include one or more. When the 2D scout image(s) include multiple 2D scout images, a plurality of scout scans may need to be performed. During each scout scan, a radiation source and a detector may be stationary, and a scanning bed may carry the target subject to move.

The image(s) captured in the scout scan(s) may be 2D scout image(s), which are similar to 2D scout images obtained by projecting a 3D image to one or more planes. Therefore, if the internal structure of the scanned subject when the 2D scout image is obtained compared with the internal structure of the scanned subject when 3D base image is obtained, the change may be revealed in the scout image(s). Based on the 2D scout image(s), the 3D base image, and a transformation relationship between the 2D scout image(s) and the 3D base image, how the internal structure of the scanned subject has changed (e.g., which physical points have changed and how do gray values of voxels corresponding to these physical points change) may be determined.

In some embodiments, when the count of the 2D scout image(s) is multiple, scanning angles corresponding to different scout images may be different. That is to say, the positions of the radiation source relative to the target subject in different scout scans may be different. For example, the 2D scout image(s) may include an anteroposterior scout image and a lateral scout image. In some embodiments, the scout scans corresponding to the plurality of 2D scout images may be performed sequentially and intervals between the scout scans may be relatively short (e.g., shorter than a preset threshold). In such cases, the plurality of 2D scout images may be deemed as being captured at the same time and reflect the internal structure of the target subject at a specific time.

In some embodiments, the posture of the target subject in a scout scan may be the same as the posture of the target subject in the 3D scan described in the operation 310, but the internal structure of the target subject in the scout scan has changed compared with the internal structure of the target subject in the 3D scan. The change of the internal structure of the target subject may be caused by multiple factors. For example, the change of the internal structure of the target subject may be caused by an intervention of an intervention equipment into the target subject. At this time, the 2D scout image(s) may include scanning image(s) captured after the intervention equipment is inserted into the target subject. As another example, the change of the internal structure of the target subject may be caused by physiological change of the ROI of the target subject. For example, the infection region of the target subject may spread, which causes a change in the infarction region and nearby regions.

In some embodiments, the processing device 120 may obtain the 2D scout image(s) of the target subject by other manners, for example, obtain the 2D scout image(s) from a storage device, etc.

In 330, a 3D predicted image of the target subject may be generated based on the 3D base image and the one or more 2D scout images. In some embodiments, the operation 330 may be executed by the predicted image generation module 230.

The 3D predicted image may display an internal structure of the target subject when the one or more 2D scout images are captured, i.e., after the internal structure of the target subject changes. For example, the 3D predicted image may reflect the internal structure of the target subject after the intervention equipment has been inserted into the target subject.

In some embodiments, prior information may be acquired and used to generate the 3D predicted image. The prior information may indicate a transformation relationship between the 3D base image, the one or more 2D scout images, and the 3D predicted image, such as a corresponding relationship among points in the 3D base image, points in each 2D scout image, and points in the 3D predicted image, etc. In some embodiments, the processing device 120 may obtain the prior information based on historical scanning data, simulated scanning data, or the like, such as based on historical intervention imaging data, based on historical examination data, based on digital phantom simulated scanning data, etc.

In some embodiments, an image generation model generated by machine learning technologies may be used to generate the 3D predicted image. In some embodiments, the image generation model may include various deep learning models, such as a Convolutional Neural Network (CNN), a Recursive Neural Network (RNN), a Deep Belief Neural Network (DBN), etc. The input of the image generation model may include the 3D base image and the one or more 2D scout images, and the output of the image generation model may include the 3D predicted image. The processing device 120 may generate the 3D predicted image by processing the 3D base image and the one or more 2D scout images using the image generation model.

In some embodiments, the processing device 120 may generate a preprocessed 3D base image and one or more preprocessed 2D scout images by preprocessing at least one of the 3D base image and the one or more 2D scout images. Further, the processing device 120 may obtain the 3D predicted image by processing the preprocessed 3D base image and the one or more preprocessed 2D scout images using the image generation model. The preprocessing may include various image processing operations, such as image noise reduction, image registration, FOV adjustment, brightness, etc. For example, the preprocessing may include adjusting the brightness of the 3D base image and each 2D scout image to a preset value. As another example, the preprocessing may include adjusting a field of view (FOV) of the 3D base image and each 2D scout image to make the preprocessed 3D base image and the one or more preprocessed 2D scout images correspond to the same FOV. In some embodiments, when the two images correspond to a same part of the target subject, the FOVs of the two images may be considered to be the same. For example, when the FOV of the 2D scout image(s) is greater than the FOV of the 3D base image, local regions corresponding to the FOV of the 3D base image may be segmented from the 2D scout image(s), or a periphery of the 3D base image may be filled.
In some embodiments, the processing device 120 may further obtain reference information, and generate the 3D predicted image based on the reference information. For example, the input of the image generation model may further include the reference information. The processing device 120 may generate the 3D predicted image by processing the 3D base image, the one or more 2D scout images, and the reference information using the image generation model. The reference information may relate to at least one of an intervention point of the intervention equipment (i.e., a point where the intervention equipment inserts into the target subject), a moving route of the intervention equipment, a shape of the intervention equipment, a material of the intervention equipment, or a type of the intervention equipment. The intervention point of the intervention equipment and the moving route of the intervention equipment may reflect a trajectory that the intervention equipment plans to move along in the target subject. The material of the intervention equipment (e.g., plastic, metal, etc.), the shape of the intervention equipment (e.g., the length, the thickness, etc.), or the type of the intervention equipment (e.g., an interventional needle, an intervention catheter) may affect artifacts (e.g., tip artifact) in the 3D predicted image. In general, different intervention equipment with different materials may cause artifacts with different strengths, and intervention equipment with different shapes may cause artifacts with different shapes. In some embodiments of the present disclosure, by using the reference information relating to the intervention equipment to generate the 3D predicted image, the quality and accuracy of the generated 3D predicted image can be improved and the generated 3D predicted image may reflect a state of the intervention equipment more accuracy, so that the doctors can determine the current progress of the intervention operation better and the intervention therapy may be performed safely and accurately. For example, based on the intervention point and the moving route of the intervention equipment, the image generation model may determine the position of the intervention equipment in the 3D predicted image with an improved accuracy. As another example, based on the information such as the material, shape, and type of the intervention equipment, the image generation model may adjust artifact correction strength adaptively and reduce the artifacts in the 3D predicted image.

The target subject has a physiological motion such as a respiratory motion. If the 2D scout image(s) and the 3D base image correspond to different physiological phases, the 3D predicted image can not reflect a current situation of the target subject accurately. Therefore, a specific measure needs to be adopted to avoid the effect of the physiological motion. The 3D base image does include a plurality of frames corresponding to the plurality of physiological phases of the target subject, such as multiple frames corresponding to a plurality of respiratory phases of the respiratory motion. The processing device 120 determines a target physiological phase of the target subject corresponding to the one or more 2D scout images (i.e., the physiological phase of the target subject when the 2D scout image(s) are captured). The processing device 120 selects, from the plurality of frames, a target frame corresponding to the target physiological phase and generates the 3D image predicted by processing the target frame and the one or more 2D scout images using the image generation model. Taking the respiratory motion as an example, the processing device 120 may determine the respiratory phase (i.e., the target physiological phase) that corresponds to the 2D scout image(s), designate an image frame that corresponds to the respiratory phase as the target frame, and obtain the 3D predicted image by inputting the 2D scout image(s) and the target frame into the image generation model for processing.

In some embodiments, to avoid the effect of the physiological motion, the physiological motion of the target subject may be monitored and the 2D scout image(s) may be captured in a physiological phase when the physiological motion is smooth. For example, the 2D scout image(s) may be captured in a middle and late diastole when the cardiac motion of the target subject is relatively smooth. The processing device 230 may generate the 3D predicted image based on the 2D scout image(s) captured when the physiological motion is smooth and the 3D base image. The 2D scout image(s) captured when the physiological motion is smooth may be regarded as being not affected by the physiological motion or the effect of the physiological motion on the 2D scout image(s) is within an acceptable range.

In some embodiments of the present disclosure, the 3D predicted image may be generated by obtaining the target frame of the 3D base image corresponding to the physiological phase of the 2D scout image(s), so that the images used to generate the 3D predicted image correspond to the same physiological phase of the target subject, and the effect of the physiological motion on the image prediction can be avoided, the obtained predicted image can reflect the current situation of the target subject accurately, which provides a reliable guarantee for the diagnosis and treatment.

In some embodiments, the image generation model may be trained using a plurality of training samples, each training sample may include a ground truth 3D image, one or more sample 2D scout images, and a sample 3D base image of the sample subject. The sample 3D base image may indicate an internal structure of a sample subject before an internal structure of the sample subject changes, the ground truth 3D scout image and the sample 2D scout images may indicate the internal structure of the sample subject after the internal structure changes. For example, the ground truth 3D image and the sample 2D scout image(s) may indicate the internal structure of the sample subject with a sample intervention equipment inside the sample subject, the sample 3D base image may indicate the internal structure of the sample subject without the sample intervention equipment inside the sample subject. As another example, the sample 3D base image may reflect the internal structure of the sample subject before an ROI (e.g., an infection region) of the sample subject has physiological change; the ground truth 3D image and the sample 2D scout image may reflect the internal structure of the sample subject after the ROI (e.g., the infection region) of the sample subject has physiological change.

Postures of the sample subject in the ground truth 3D image, the sample 2D scout image(s), and the sample 3D base image may be the same or basically the same. During model training, the ground truth 3D image may be used as a training label, the one or more sample 2D scout images and the sample 3D base image may be used as a model input. The sample intervention equipment may include a sample interventional needle, a sample intervention catheter, or the like. In some embodiments, the sample intervention equipment and the above intervention equipment may be the intervention equipment of the same type. The sample subject may be a subject of the same type as the above target subject, such as sample patients. For example, a training sample may include: a ground truth 3D image and at least one sample 2D scout image indicating an internal structure of a sample patient when a sample interventional needle has been inserted into the sample patient, and a sample 3D base image indicating the internal structure of the sample patient when the sample interventional needle has not been inserted into the sample patient.

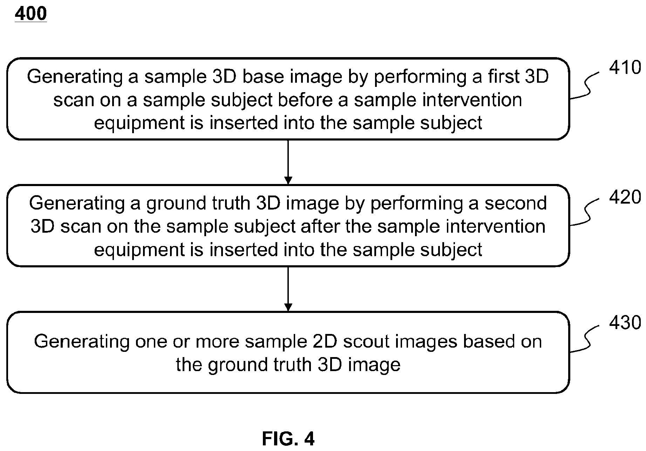

In some embodiments, the processing device 120 may obtain the training samples by multiple manners. For example, the sample images (e.g., the ground truth 3D image, the sample 2D scout image(s), the sample 3D base image) used for training may be real captured images, images generated after processing the real captured images, and simulated images generated based on a digital model. In some embodiments, in order to obtain enough samples for improving the quality of the model training, the sample 2D scout image(s) may include images captured by a plurality of angles, for example, the sample 2D scout image(s) may include images captured by at least six angles. In some embodiments, for clearly showing the intervention equipment in the sample 2D scout image(s), an imaging plane of the sample 2D scout image(s) may not be perpendicular to a moving direction of the intervention equipment. That is to say, when capturing the sample 2D scout image(s), a line connecting the radiation source and the detector (real radiation source and real detector or simulated radiation source and simulated detector) may not be in a direction of the moving direction or a reverse direction of the moving direction of the intervention equipment. More descriptions regarding how to obtain the training samples of the image generation model may be found elsewhere in the present disclosure. See, e.g., FIGs. 4-6 and relevant descriptions thereof.

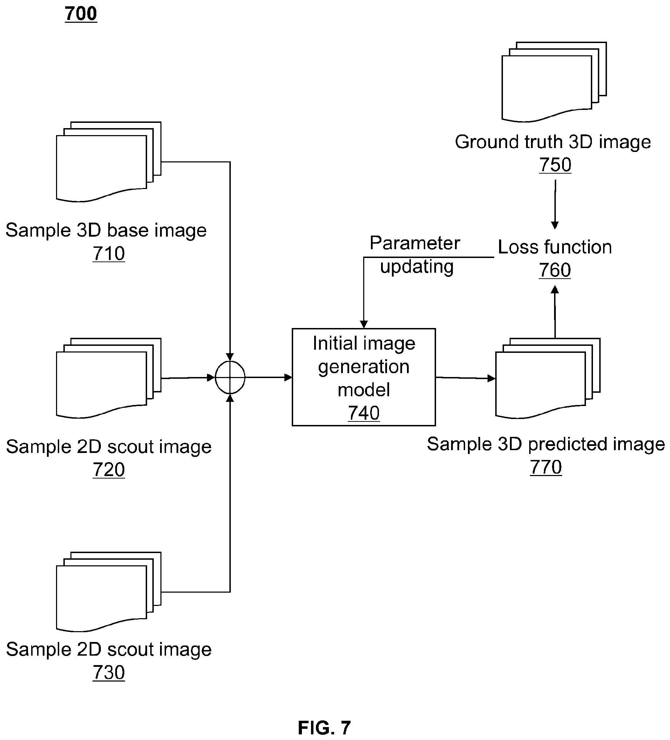

In some embodiments, the processing device 120 may obtain the trained image generation model by training an initial image generation model using the training samples. More descriptions regarding a generating process of the image generation model may be found elsewhere in the present disclosure. See, e.g., FIGs. 7-8 and relevant descriptions thereof.

In some embodiments, the processing device 120 may reduce or eliminate tip artifacts by performing artifact correction on the generated 3D predicted image. For example, the processing device 120 may generate an initial 3D predicted image by processing the 3D base image and the one or more 2D scout images using the image generation model, and generate the 3D predicted image by performing the artifact correction on the generated initial 3D predicted image.

In some embodiments, the processing device 120 may perform the artifact correction using an artifact correction model. The artifact correction model may include various deep learning model, such as a Convolutional Neural Network (CNN), a Recursive Neural Network (RNN), a Deep Belief Neural Network (DBN), or the like. In some embodiments, an input of the artifact correction model may include the initial 3D predicted image generated by the image generation model, an output may include the 3D prediction image after tip artifact removal.

In some embodiments, training samples of the artifact correction model may include a first sample 3D image and a second sample 3D image, wherein the first sample 3D image has not undergone artifact correction, and at least a portion of artifacts in the second sample 3D image has been removed. The second sample 3D image may be used as a training label, the first sample 3D image may be used as a model input. In some embodiments, the first sample 3D image may be obtained by performing a real scan. In some embodiments, the second sample 3D image used as the training label may be obtained by fusing a pure tissue image with a pure intervention image, or the second sample 3D image may be obtained by performing the correction on the first sample 3D image using artifact correction algorithms.

In some embodiments, the artifact correction model may be trained independently. The processing device 120 may obtain a predicted correction image by inputting the first sample 3D image into an initial artifact correction model, determine a loss function based on the predicted correction image and the second sample 3D image, iteratively update the initial artifact correction model based on the loss function until the trained artifact correction model is obtained.

In some embodiments, the artifact correction model may be trained jointly with the image generation model. In some embodiments, the training samples used in the joint training process may include: a sample 3D base image of the sample subject, a sample ground truth 3D image, and sample 2D scout image(s). The sample ground truth 3D image may be an image without tip artifacts (e.g., an image without tip artifacts generated by simulate manners, an image obtained by performing artifact correction on a real captured image, etc.). The processing device 120 may input the sample 3D base image and the sample 2D scout image(s) into the initial image generation model, the initial image generation model may output the initial 3D predicted image; the processing device 120 may input the initial 3D predicted image into the initial artifact correction model, the initial artifact correction model may output a target 3D predicted image. The processing device 120 may determine a value of a loss function based on the target 3D predicted image and the sample ground truth 3D image. The value of the loss function may be used to update the initial image generation model and the initial artifact correction model until a termination condition is satisfied.

In some embodiments of the present disclosure, by performing artifact correction on the obtained 3D predicted image using a deep learning model, the tip artifacts may be removed or reduced, and the predicted image quality may be further improved. In some embodiments, the artifact correction model and the image generation model may be generated by joint training, which can improve the efficiency of the model training and reduce the training time.

In some embodiments, the operations 320 and 330 may be performed multiple times to observe the internal structure of the target subject continuously. For example, during the interventional therapy, the 2D scout image(s) and the 3D predicted image corresponding to different times may be obtained by performing the operations 320 and 330 multiple times, to track the real-time position of the intervention equipment in the target subject, which can improve the accuracy of the interventional therapy. As another example, during the repeat examination, the 2D scout image(s) and the 3D predicted image corresponding to different examinations may be obtained by performing the operations 320 and 330 multiple times, to track the change of the lesion region continuously.

FIG. 4 is a flowchart illustrating an exemplary process for generating a training samples of an image generation model according to some embodiments of the present disclosure.

In some embodiments, the process 400 may be executed by the model training module of the processing device 120. As shown in FIG. 4, the process 400 may include the following operations:

In 410, a sample 3D base image may be generated by performing a first 3D scan on the sample subject before a sample intervention equipment is inserted into the sample subject.

For example, before the sample intervention equipment (i.e., an interventional equipment used as a sample) is inserted into the sample subject, the processing device 120 may instruct a CT device to perform the first 3D scan on the sample subject, and use the obtained 3D scanning image as the sample 3D base image. The sample 3D base image captured by the first 3D scan may be a real image, which reflects an internal structure state of the sample subject when the sample intervention equipment is not inserted into the sample subject. The sample subject may be similar to the target subject described in FIG. 3. For example, the sample subject may be a specific body region of a sample patient, such as the brain, the heart, the stomach, etc.

In some embodiments, the processing device 120 may obtain the sample 3D base image from a storage device.

In 420, a ground truth 3D image may be generated by performing a second 3D scan on the sample subject after the sample intervention equipment is inserted into the sample subject.

For example, after the sample intervention equipment is inserted into the sample subject, the processing device 120 may instruct the CT device to perform the second 3D scan and use the obtained 3D scan image as the ground truth 3D image. The region of the sample subject scanned in the first 3D scan and the second 3D scan may be the same, and the posture of the sample subject in the first 3D scan may be the same as the posture of sample subject in the second 3D scan. The ground truth 3D image captured by the second 3D scan may be a real image, which reflects an internal structure state of the sample subject after the intervention equipment is inserted into the sample subject. In some embodiments, a preliminary image may be obtained by the second 3D image, and the preliminary image may be processed to generate the ground truth 3D image. For example, artifact correction may be performed on the preliminary image to generate the ground truth 3D image.

In 430, the one or more sample 2D scout images may be generated based on the ground truth 3D image.

In some embodiments, the processing device 120 may project the ground truth 3D image in at least one direction and use the obtained projected image(s) as the sample 2D scout image(s). For example, anteroposterior and lateral sample 2D scout images may be obtained by projecting the ground truth 3D image from the front and the side of the sample subject.

In some embodiments, before the sample 2D scout image(s) are generated, the processing device 120 may preprocess the ground truth 3D image, for example, crop the ground truth 3D image, remove tip artifacts from the ground truth 3D image, etc.

In some embodiments of the present disclosure, the sample base image and the ground truth 3D image obtained by real scans may be used as the training sample of the image generation model, the image generation model may learn an optimal mechanism of generating a real-time 3D image based on real-time 2D image(s) and a 3D base image from real images, so that predicted images generated by the image generation model may be closer to actual images and the performance of the trained model can be improved.

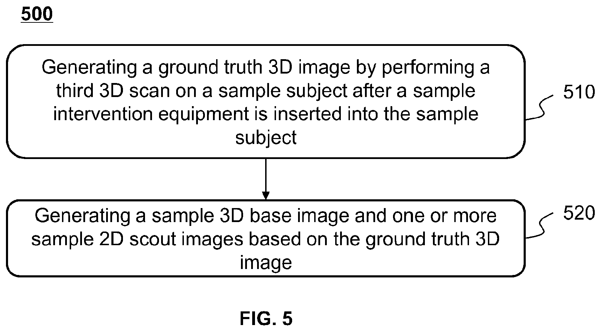

FIG. 5 is a flowchart illustrating an exemplary process for generating a training sample of an image generation model according to some embodiments of the present disclosure.

In some embodiments, the process 500 may be executed by the model training module of the processing device 120. As shown in FIG. 5, the process 500 may include the following operations.

In 510, a ground truth 3D image may be generated by performing a third 3D scan on a sample subject after a sample intervention equipment is inserted into the sample subject.

The third scan may be similar to the second 3D scan described in the operation 420. Operation 510 may be performed in a similar manner as operation 420, and the descriptions thereof are not repeated here.

In 520, a sample 3D base image and one or more sample 2D scout images may be generated based on the ground truth 3D image.

In some embodiments, the processing device 120 may project the ground truth 3D image in at least one direction and use the obtained projected image(s) as the sample 2D scout image(s). More descriptions regarding the generation of the sample 2D scout image(s) based on the ground truth 3D image may be found elsewhere in the present disclosure. See, e.g., operation 430 and relevant descriptions thereof.

In some embodiments, the processing device 120 may obtain the sample 3D base image by processing (e.g., segmenting, repairing, etc.) the ground truth 3D image. For example, the processing device 120 may perform segmentation and tissue repair (e.g., through interpolation and other processing manners) on voxels of the sample subject that have changed due to the intervention of a sample interventional equipment, and obtain the sample 3D base image indicating the internal structure of the sample subject before the internal structure changes (e.g., before the sample interventional equipment is inserted into the sample subject).

In some embodiments, the processing device 120 may generate the sample 3D base image based on the ground truth 3D image using a second image generation model. For example, the processing device 120 may train the second image generation model using training images of sample subjects captured with/without an interventional needle, and the second image generation model may be used to generate an image without the interventional needle based on an image with the interventional needle.

In some embodiments of the present disclosure, the ground truth 3D image when the intervention equipment is inserted into the sample subject may be captured by a real scan, and other training images of the image generation model may be obtained based on the ground truth 3D image, which can reduce the image captured times and the radiation doses received by the doctors and the sample subject, and protect the health of the doctors and the sample subject. The 3D image of a sample subject after the internal structure changes may be easier to obtain than a 3D image of the sample subject before the internal structure changes. Therefore, the training sample obtaining manner in the process 500 may be relatively easy to implement.

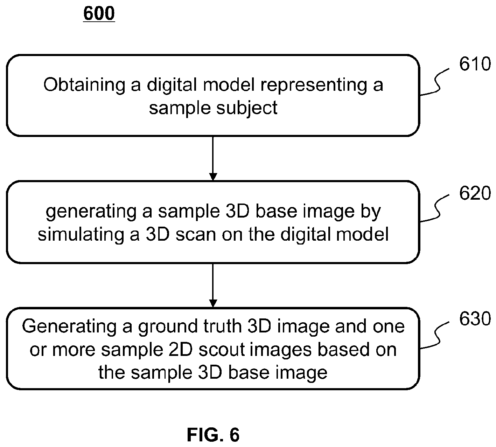

FIG. 6 is a flowchart illustrating an exemplary process for generating a training sample of an image generation model according to some embodiments of the present disclosure.

In some embodiments, the process 600 may be executed by the model training module of the processing device 120. As shown in FIG. 6, the process 600 may include the following operations.

In 610, a digital model representing a sample subject may be obtained.

The digital model of the sample subject may store data relating to a body structure of the sample subject in a digital form, such as boundary data of different organs, element compositions, attenuation characteristics with respect to X-rays, or the like. In some embodiments, the processing device 120 may simulate and generate the digital model of the sample subject according to a standard structure of human body. In some embodiments, the processing device 120 may obtain structure information of the sample subject based on a scanning image of the sample subject (e.g., a CT image, etc.), and generate the digital model of the sample subject according to the structure information of the sample subject.

In 620, a sample 3D base image may be generated by simulating a 3D scan on the digital model.

In some embodiments, the processing device 120 may simulate a transmission process of medical scanning (e.g., CT scanning) rays in the sample subject based on the structure information of the sample subject contained in the digital model, and generate the sample 3D base image by performing image reconstruction on the scanning data obtained by the simulated scan. The sample 3D base image may be a simulated image, which reflects an internal structure state of the sample subject before the internal structure changes (i.e., before a sample intervention equipment is inserted into the sample subject).

In 630, a ground truth 3D image and one or more sample 2D scout images may be generated based on the sample 3D base image.

In some embodiments, the processing device 120 may obtain information relating to factors leading to the change of the internal structure of the sample subject (e.g., images of a sample interventional needle, images of an infection region of the sample subject, etc.), and obtain the ground truth 3D image by fusing the information with the sample 3D base image. The processing device 120 may obtain the sample 2D scout image(s) by projecting the ground truth 3D image.

For example, the processing device 120 may obtain images of a sample interventional needle (an image without any artifact), generate images of the sample interventional needle with various states according to motion information of the sample interventional needle, and obtain ground truth 3D images corresponding to the sample interventional needle at different positions and with different angles by fusing these images with the sample 3D base image. The processing device 120 may obtain the corresponding sample 2D scout image(s) by projecting the ground truth 3D images.

In some embodiments, the processing device 120 may determine different positions of the sample interventional needle during a simulation process according to characteristics of the sample interventional needle. For example, when performing an abdominal liver puncture, the different positions of the sample interventional needle may be obtained by simulating the insertion of the sample interventional needle into the liver from different positions with different depths. Rich training data set may be obtained by data simulation, so that an image generation network can be trained more effectively.

As another example, the processing device 120 may obtain images of the infection region of the sample subject at different change stages. The ground truth 3D images corresponding to the infection region at different change stages may be obtained by fusing the images with the sample 3D base image. Then, the processing device 120 may obtain the corresponding sample 2D scout image(s) by projecting the ground truth 3D images.

In some embodiments of the present disclosure, simulated images generated based on the digital model of the sample subject may be used as the training samples, which does not need to capture actual images before and after the internal structure of the sample subject changes, so as to eliminate the radiation effect received by the doctors and the sample subject. In addition, the simulated ground truth 3D image do not include the artifacts, so the 3D predicted image generated by the generated image generation model in the application also does not contain artifacts, and no additional correction operation is required. Furthermore, a large number of training data may be obtained in a short time through data simulation, and the training data may be highly customized (e.g., training data corresponding to interventional needles with different sizes and materials may be generated), which can improve the training efficiency and quality of the image generation model.

It should be noted that the above processes 300, 400, 500, and 600 are merely provided for the purposes of illustration, and not intended to limit the scope of the present disclosure. For persons having ordinary skills in the art, multiple variations and modifications for the processes 300, 400, 500, and 600 may be made under the teachings of the present disclosure. However, those variations and modifications do not depart from the scope of the present disclosure. For example, the sequence of the process 410 and the process 420 may be changed. As another example, the training samples of the image generation model may be generated by performing two or more of the processes 400, 500, and 600.

In addition, it should be noted that FIGs. 4 and 5 illustrate the obtaining process of the sample 3D base image and the ground truth 3D image with interventional therapy as an example. In some embodiments, the sample 3D base image may reflect the internal structure of the sample subject before the physiological change of an ROI (e.g., an infection region) of the sample subject; the ground truth 3D image and the sample 2D scout image(s) may reflect the internal structure of the sample subject after the physiological change of the ROI.

FIG. 7 is a schematic diagram illustrating an exemplary method 700 for training an image generation model according to some embodiments of the present disclosure.

As shown in FIG. 7, during the training process, the input of an initial image generation model 740 may include a sample 3D base image 710, a sample 2D scout image 720, and a sample 2D scout image 730. The sample 3D base image 710 may be a 3D scanning image captured before the internal structure of the sample subject changes, such as a 3D scanning image of the sample subject captured when a sample interventional needle has not been inserted into the sample subject. The sample 2D scout image 720 and the sample 2D scout image 730 may be 2D scout scanning images captured at different scanning angles after the internal structure of the sample subject changes.

The processing device 120 may obtain a sample 3D predicted image 770 by inputting the sample 3D base image 710, the sample 2D scout image 720, and the sample 2D scout image 730 into the initial image generation model 740; obtain a value of a loss function 760 by comparing the sample 3D predicted image 770 and the ground truth 3D image 750; update the parameters of the initial image generation model 740 based on the value of the loss function 760. In some embodiments, the above process may be performed repeatedly until a model training termination condition is satisfied, for example, a specific number of iterations is performed, the value of the loss function 760 convergences, the value of the loss function 760 is less than a preset threshold, or the like. When the model training termination condition is satisfied, the updated image generation model 740 may be designated as the image generation model.

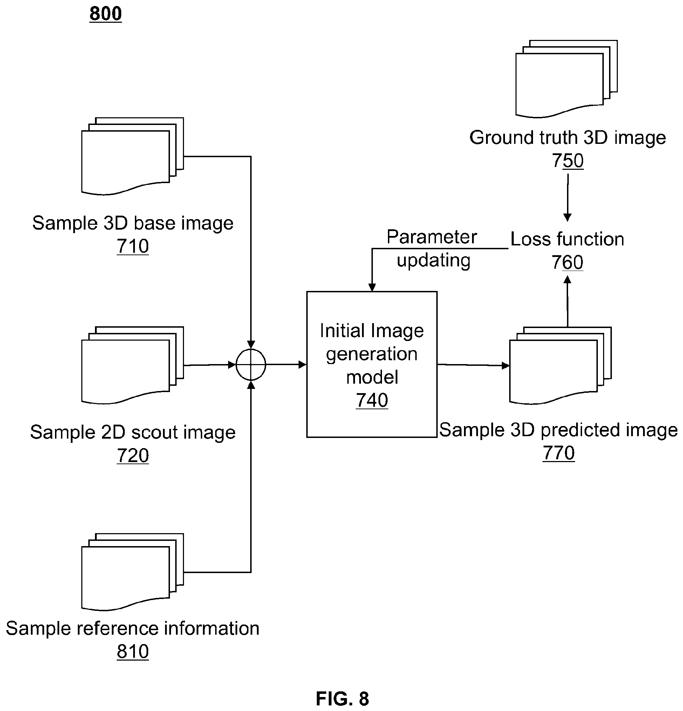

FIG. 8 is a schematic diagram illustrating another exemplary method 800 for training an image generation model according to some embodiments of the present disclosure.

The method 800 illustrated in FIG. 8 may be similar to the method 700 illustrated in FIG. 7, except that the input of the initial image generation model 740 may include the sample 3D base image 710, the sample 2D scout image 720, and sample reference information 810. The sample reference information 810 may be similar to the reference information described in FIG. 3. For example, the sample reference information 810 may include at least one of the intervention point of a sample intervention equipment, the moving route of the sample intervention equipment, the shape of the sample intervention equipment, the material of the sample intervention equipment, or the type of the sample intervention equipment. In some embodiments, the initial image generation model 740 may determine artifacts based on the sample reference information 810. For example, at least one of a size, shape, and position of the artifacts may be determined based on the sample reference information 810. In some embodiments, the plurality of image generation models may be trained based on the sample reference information 810. For example, an image generation model corresponding to a metal interventional needle and an image generation model corresponding to a plastic interventional needle may be trained.

The possible beneficial effects of the embodiments of the present disclosure may include but may not be limited to: (1) an image generation model may be generated in advance, and the predicted 3D image may be generated in real time using the image generation model based on the 3D base image and 2D scout image(s), which can improve the imaging speed; (2) the 2D scout image(s) may be captured by performing a scout scan with relatively low radiation. Compared with capturing the predicted 3D image by performing a 3D scan repeatedly, the predicted 3D image generated based on the 2D scout image(s) can reduce the time that doctors and patients are exposed to a radiation field, greatly reduce scanning doses received by the doctors and patients, and ensures the health of the doctors and patients; (3) a gold standard image used for training the image generation model (i.e., a ground truth 3D image) may be generated by data simulation manners such as using a digital model or correcting a real image, the image generation model may learn image prediction mechanisms based on the gold standard image without artifacts or with relatively less artifacts. Therefore, the 3D predicted image generated by the image generation model during application does not contain artifacts or has fewer artifacts, which can improve the quality of the 3D predicted image. It should be noted that different embodiments may produce different beneficial effects. In different embodiments, the beneficial effects may be any one or a combination of the above, or any other beneficial effects that may be obtained.

The basic concepts have been described. Obviously, for those skilled in the art, the detailed disclosure may be only an example and may not constitute a limitation to the present disclosure. Although not explicitly stated here, those skilled in the art may make various modifications, improvements, and amendments to the present disclosure. These alterations, improvements, and modifications are intended to be suggested by this disclosure and are within the scope of the exemplary embodiments of this disclosure.

Moreover, certain terminology has been used to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of the specification are not necessarily all referring to the same embodiment. In addition, some features, structures, or features in the present disclosure of one or more embodiments may be appropriately combined.

Moreover, unless otherwise specified in the claims, the sequence of the processing elements and sequences of the present application, the use of digital letters, or other names are not used to define the order of the application flow and methods. Although the above disclosure discusses through various examples what is currently considered to be a variety of useful embodiments of the disclosure, it is to be understood that such detail is solely for that purpose and that the appended claims are not limited to the disclosed embodiments, but, on the contrary, are intended to cover modifications and equivalent arrangements that are within the scope of the disclosed embodiments. For example, although the implementation of various assemblies described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure and aiding in the understanding of one or more of the various embodiments. However, this disclosure may not mean that the present disclosure object requires more features than the features mentioned in the claims. In fact, the features of the embodiments are less than all of the features of the individual embodiments disclosed above.

In some embodiments, numbers describing the number of ingredients and attributes are used. It should be understood that such numbers used for the description of the embodiments use the modifier "about", "approximately", or "substantially" in some examples. Unless otherwise stated, "about", "approximately", or "substantially" indicates that the number is allowed to vary by ±20%. Correspondingly, in some embodiments, the numerical parameters used in the description and claims are approximate values, and the approximate values may be changed according to the required characteristics of individual embodiments. In some embodiments, the numerical parameters should consider the prescribed effective digits and adopt the method of general digit retention. Although the numerical ranges and parameters used to confirm the breadth of the range in some embodiments of the present disclosure are approximate values, in specific embodiments, settings of such numerical values are as accurate as possible within a feasible range.

For each patent, patent application, patent application publication, or other materials cited in the present disclosure, such as articles, books, specifications, publications, documents, or the like, the entire contents of which are hereby incorporated into the present disclosure as a reference. The application history documents that are inconsistent or conflict with the content of the present disclosure are excluded, and the documents that restrict the broadest scope of the claims of the present disclosure (currently or later attached to the present disclosure) are also excluded. It should be noted that if there is any inconsistency or conflict between the description, definition, and/or use of terms in the auxiliary materials of the present disclosure and the content of the present disclosure, the description, definition, and/or use of terms in the present disclosure is subject to the present disclosure.

At last, it should be understood that the embodiments described in the disclosure are used only to illustrate the principles of the embodiments of this application. Other modifications may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described, but are only limited by the appended claims.

## Claims

1. A method for image generation, implemented on a computing device having one or more processors and one or more storage devices, the method comprising the steps of:
obtaining (310) a three-dimensional, 3D, base image of a target subject, the 3D base image being captured by performing a 3D scan on the target subject with a preset posture, the 3D base image including a plurality of frames corresponding to a plurality of physiological phases of the target subject;
obtaining (320) one or more two-dimensional, 2D, scout images of the target subject, the one or more 2D scout images of the target subject being captured by performing a scout scan on the target subject with the preset posture after an internal structure of the target subject changes;
determining a target physiological phase of the target subject corresponding to the one or more 2D scout images;
selecting, from the plurality of frames, a target frame corresponding to the target physiological phase;
generating (330) a 3D predicted image of the subject by processing the target frame and the one or more 2D scout images using an image generation model, the 3D predicted image indicating the internal structure of the target subject when the one or more 2D scout images are captured, wherein the image generation model is a machine learning model.

2. The method of claim 1, wherein the one or more 2D scout images are obtained by:
monitoring the physiological motion of the target subject;
in response to detecting that the target subject is in a middle and late diastole when the cardiac motion of the target subject is smooth, performing the scout scan to capture the one or more 2D scout images of the target subject.

3. The method of claim 1 or 2, wherein the internal structure of the target subject changes due to at least one of an intervention of an intervention equipment into the target subject or physiological change of a region of interest of the target subject.

4. The method of claim 3, the method further comprising:
obtaining reference information relating to at least one of an intervention point of the intervention equipment, a moving route of the intervention equipment, a shape of the intervention equipment, a material of the intervention equipment, or a type of the intervention equipment, wherein the 3D predicted image is generated further based on the reference information.

5. The method of claim 3 or 4, wherein the image generation model is trained using a plurality of training samples, each of which includes:
a ground truth 3D image and one or more sample 2D scout images indicating the internal structure of a sample subject with a sample intervention equipment inside the sample subject, and
a sample 3D base image indicating the internal structure of the sample subject without the sample intervention equipment inside the sample subject, wherein the target subject is a patient that needs to be treated or examined, the sample subject is a sample patient.

6. The method of claim 5, wherein a training sample of the plurality of training samples is generated by:
generating (410) the sample 3D base image by performing a first 3D scan on the sample subject before the sample intervention equipment is inserted into the sample subject;
generating (420) the ground truth 3D image by performing a second 3D scan on the sample subject after the sample intervention equipment is inserted into the sample subject; and
generating (430) the one or more sample 2D scout images based on the ground truth 3D image.

7. The method of claim 5, wherein a training sample of the plurality of training samples is generated by:
generating (510) the ground truth 3D image by performing a third 3D scan on the sample subject after the sample intervention equipment is inserted into the sample subject; and
generating (520) the sample 3D base image and the one or more sample 2D scout images based on the ground truth 3D image.

8. The method of claim 7, wherein the sample 3D base image is generated based on the ground truth 3D image using a second image generation model, the second image generation model being trained using a training image of the sample subject captured with the intervention equipment and a training image of the sample subjects captured without the intervention equipment.

9. The method of claim 5, wherein a training sample of the plurality of training samples is generated by:
obtaining (610) a digital model representing the sample subject;
generating (620) the sample 3D base image by simulating a 3D scan on the digital model; and
generating (630) the ground truth 3D image and the one or more sample 2D scout images based on the sample 3D base image.

10. The method of claim 1, wherein the generating the 3D predicted image by processing the target frame and the one or more 2D scout images using the image generation model comprises:
generating a preliminary 3D predicted image by processing the target frame and the one or more 2D scout images using the image generation model; and
generating the 3D predicted image by performing artifact correction on the preliminary 3D predicted image using an artifact correction model, the artifact correction model being jointly trained with the image generation model.

11. The method of any one of claims 1 to 10, wherein the one or more scout images include multiple scout images corresponding to different scanning angles.

12. A system for image generation, implemented on a computing device having one or more processors and one or more storage devices, the system comprising:
at least one storage device including a set of instructions; and
at least one processor configured to communicate with the at least one storage device, wherein when executing the set of instructions, the at least one processor is configured to direct the system to perform operations including:
obtaining (310) a three-dimensional, 3D, base image of a target subject, the 3D base image being captured by performing a 3D scan on the target subject with a preset posture, the 3D base image including a plurality of frames corresponding to a plurality of physiological phases of the target subject;
obtaining (320) one or more two-dimensional, 2D, scout images of the target subject, the one or more 2D scout images of the target subject being captured by performing a scout scan on the target subject with the preset posture after an internal structure of the target subject changes;
determining a target physiological phase of the target subject corresponding to the one or more 2D scout images;
selecting, from the plurality of frames, a target frame corresponding to the target physiological phase;
generating (330) a 3D predicted image of the subject by processing the target frame image and the one or more 2D scout images using an image generation model, the 3D predicted image indicating the internal structure of the target subject when the one or more 2D scout images are captured, wherein the image generation model is a machine learning model.

13. The system of claim 12, wherein the one or more 2D scout images are obtained by:
monitoring the physiological motion of the target subject;
in response to detecting that the target subject is in a middle and late diastole when the cardiac motion of the target subject is smooth, performing the scout scan to capture the one or more 2D scout images of the target subject.

14. The system of claim 12 or 13, wherein the internal structure of the target subject changes due to at least one of an intervention of an intervention equipment into the target subject or physiological change of a region of interest of the target subject.

15. A non-transitory computer readable medium, comprising at least one set of instructions, wherein when executed by one or more processors of a computing device, the at least one set of instructions causes the computing device to perform a method, the method comprising:
obtaining (310) a three-dimensional, 3D, base image of a target subject, the 3D base image being captured by performing a 3D scan on the target subject with a preset posture, the 3D base image including a plurality of frames corresponding to a plurality of physiological phases of the target subject;
obtaining (320) one or more two-dimensional, 2D, scout images of the target subject, the one or more 2D scout images of the target subject being captured by performing a scout scan on the target subject with the preset posture after an internal structure of the target subject changes;
determining a target physiological phase of the target subject corresponding to the one or more 2D scout images;
selecting, from the plurality of frames, a target frame corresponding to the target physiological phase;
generating (330) a 3D predicted image of the subject by processing the target frame and the one or more 2D scout images using an image generation model, the 3D predicted image indicating the internal structure of the target subject when the one or more 2D scout images are captured, wherein the image generation model is a machine learning model.

## Patentansprüche

1. Verfahren zur Bilderzeugung, das auf einer Rechenvorrichtung implementiert ist, die einen oder mehrere Prozessoren und eine oder mehrere Speichervorrichtungen aufweist, wobei das Verfahren die folgenden Schritte umfasst:
Erhalten (310) eines dreidimensionalen, 3D-, Basisbildes eines Zielobjekts, wobei das 3D-Basisbild durch Durchführen eines 3D-Scans an dem Zielobjekt in einer voreingestellten Haltung erfasst wird und das 3D-Basisbild eine Vielzahl von Einzelbildern beinhaltet, die einer Vielzahl von physiologischen Phasen des Zielobjekts entsprechen;
Erhalten (320) eines oder mehrerer zweidimensionaler, 2D-, Erkundungsbilder des Zielobjekts, wobei das eine oder die mehreren 2D-Erkundungsbilder des Zielobjekts durch Durchführen eines Erkundungsscans am Zielobjekt mit der voreingestellten Haltung aufgenommen werden, nachdem sich eine innere Struktur des Zielobjekts geändert hat;
Bestimmen einer physiologischen Zielphase des Zielobjekts entsprechend dem einen oder den mehreren 2D-Erkundungsbildern;
Auswählen eines Zieleinzelbildes aus der Vielzahl von Einzelbildern, das der physiologischen Zielphase entspricht;
Erzeugen (330) eines vorhergesagten 3D-Bildes des Objekts durch Verarbeiten des Zieleinzelbildes und des einen oder der mehreren 2D-Erkundungsbilder unter Verwendung eines Bilderzeugungsmodells, wobei das vorhergesagte 3D-Bild die innere Struktur des Zielobjekts anzeigt, wenn das eine oder die mehreren 2D-Erkundungsbilder aufgenommen werden, wobei das Bilderzeugungsmodell ein Maschinenlernmodell ist.

2. Verfahren nach Anspruch 1, wobei das eine oder die mehreren 2D-Erkundungsbilder wie folgt erhalten werden:
Überwachen der physiologischen Bewegung des Zielobjekts;
als Reaktion auf Erkennen, dass sich das Zielobjekt in einer mittleren und späten Diastole befindet, wenn die Herzbewegung des Zielobjekts gleichmäßig ist, Durchführen des Erkundungsscans, um das eine oder die mehreren 2D-Erkundungsbilder des Zielobjekts aufzunehmen.

3. Verfahren nach Anspruch 1 oder 2, wobei sich die innere Struktur des Zielobjekts aufgrund mindestens einem von einer Intervention einer Interventionsausrüstung in das Zielobjekt oder einer physiologischen Veränderung eines Interessenbereichs des Zielobjekts ändert.

4. Verfahren nach Anspruch 3, wobei das Verfahren weiter umfasst:
Erhalten von Referenzinformationen bezüglich mindestens eines von einem Interventionspunkt der Interventionsausrüstung, einer Bewegungsroute der Interventionsausrüstung, einer Form der Interventionsausrüstung, einem Material der Interventionsausrüstung oder einem Typ der Interventionsausrüstung, wobei das vorhergesagte 3D-Bild weiter auf der Grundlage der Referenzinformationen erzeugt wird.

5. Verfahren nach Anspruch 3 oder 4, wobei das Bilderzeugungsmodell unter Verwendung einer Vielzahl von Trainingsproben trainiert wird, von denen jedes beinhaltet:
ein Ground-Truth-3D-Bild und ein oder mehrere 2D-Probenerkundungsbilder, die die innere Struktur eines Probenobjekts mit einer Probeninterventionsausrüstung im Inneren des Probenobjekts zeigen, und
ein 3D-Probenbasisbild, das die innere Struktur des Probenobjekts ohne die Probeninterventionsausrüstung im Inneren des Probenobjekts zeigt, wobei das Zielobjekt ein Patient ist, der behandelt oder untersucht werden muss, das Probenobjekt ein Probenpatient ist.

6. Verfahren nach Anspruch 5, wobei eine Trainingsprobe aus der Vielzahl von Trainingsproben wie folgt erzeugt wird:
Erzeugen (410) des 3D-Probenbasisbildes durch Durchführen eines ersten 3D-Scans am Probenobjekt, bevor die Probeninterventionsausrüstung in das Probenobjekt eingeführt wird;
Erzeugen (420) des Ground-Truth-3D-Bildes durch Durchführen eines zweiten 3D-Scans am Probenobjekt, nachdem die Probeninterventionsausrüstung in das Probenobjekt eingeführt worden ist; und
Erzeugen (430) des einen oder der mehreren 2D-Probenerkundungsbilder auf der Grundlage des Ground-Truth-3D-Bildes.

7. Verfahren nach Anspruch 5, wobei eine Trainingsprobe aus der Vielzahl von Trainingsproben wie folgt erzeugt wird:
Erzeugen (510) des Ground-Truth-3D-Bildes durch Durchführen eines dritten 3D-Scans am Probenobjekt, nachdem die Probeninterventionsausrüstung in das Probenobjekt eingeführt worden ist; und
Erzeugen (520) des 3D-Probenbasisbildes und des einen oder der mehreren 2D-Probenerkundungsbilder auf der Grundlage des Ground-Truth-3D-Bildes.

8. Verfahren nach Anspruch 7, wobei das 3D-Probenbasisbild auf der Grundlage des Ground-Truth-3D-Bildes unter Verwendung eines zweiten Bilderzeugungsmodells erzeugt wird, wobei das zweite Bilderzeugungsmodell unter Verwendung eines Trainingsbildes des mit der Interventionsausrüstung aufgenommenen Probenobjekts und eines Trainingsbildes des ohne die Interventionsausrüstung aufgenommenen Probenobjekts trainiert wird.

9. Verfahren nach Anspruch 5, wobei die Trainingsprobe aus der Vielzahl von Trainingsproben wie folgt erzeugt wird:
Erhalten (610) eines digitalen Modells, das das Probenobjekt darstellt;
Erzeugen (620) des 3D-Probenbasisbildes durch Simulieren eines 3D-Scans auf dem digitalen Modell; und
Erzeugen (630) des Ground-Truth-3D-Bildes und des einen oder der mehreren 2D-Probenerkundungsbilder auf der Grundlage des 3D-Probenbasisbildes.

10. Verfahren nach Anspruch 1, wobei das Erzeugen des vorhergesagten 3D-Bildes durch Verarbeiten des Zieleinzelbildes und des einen oder der mehreren 2D-Erkundungsbilder unter Verwendung des Bilderzeugungsmodells umfasst:
Erzeugen eines vorläufigen vorhergesagten 3D-Bildes durch Verarbeiten des Zieleinzelbildes und des einen oder der mehreren 2D-Erkundungsbilder unter Verwendung des Bilderzeugungsmodells; und
Erzeugen des vorhergesagten 3D-Bildes durch Durchführen einer Artefaktkorrektur an dem vorläufigen vorhergesagten 3D-Bild unter Verwendung eines Artefaktkorrekturmodells, wobei das Artefaktkorrekturmodell gemeinsam mit dem Bilderzeugungsmodell trainiert wird.

11. Verfahren nach einem der Ansprüche 1 bis 10, wobei das eine oder die mehreren Erkundungsbilder mehrere Erkundungsbilder beinhalten, die unterschiedlichen Scanwinkeln entsprechen.

12. System zur Bilderzeugung, das auf einer Rechenvorrichtung implementiert ist, die einen oder mehrere Prozessoren und eine oder mehrere Speichervorrichtungen aufweist, wobei das System umfasst:
mindestens eine Speichervorrichtung, die einen Satz von Anweisungen beinhaltet; und
mindestens einen Prozessor, der konfiguriert ist, mit der mindestens einen Speichervorrichtung zu kommunizieren, wobei beim Ausführen des Satzes von Anweisungen der mindestens eine Prozessor konfiguriert ist, das System anzuweisen, Operationen durchzuführen, die beinhalten:
Erhalten (310) eines dreidimensionalen, 3D-, Basisbildes eines Zielobjekts, wobei das 3D-Basisbild durch Durchführen eines 3D-Scans an dem Zielobjekt in einer voreingestellten Haltung erfasst wird und das 3D-Basisbild eine Vielzahl von Einzelbildern beinhaltet, die einer Vielzahl von physiologischen Phasen des Zielobjekts entsprechen;
Erhalten (320) eines oder mehrerer zweidimensionaler, 2D-, Erkundungsbilder des Zielobjekts, wobei das eine oder die mehreren 2D-Erkundungsbilder des Zielobjekts durch Durchführen eines Erkundungsscans am Zielobjekt mit der voreingestellten Haltung aufgenommen werden, nachdem sich eine innere Struktur des Zielobjekts geändert hat;
Bestimmen einer physiologischen Zielphase des Zielobjekts entsprechend dem einen oder den mehreren 2D-Erkundungsbildern;
Auswählen eines Zieleinzelbildes aus der Vielzahl von Einzelbildern, das der physiologischen Zielphase entspricht;
Erzeugen (330) eines vorhergesagten 3D-Bildes des Objekts durch Verarbeiten des Zieleinzelbildes und des einen oder der mehreren 2D-Erkundungsbilder unter Verwendung eines Bilderzeugungsmodells, wobei das vorhergesagte 3D-Bild die innere Struktur des Zielobjekts anzeigt, wenn das eine oder die mehreren 2D-Erkundungsbilder aufgenommen werden, wobei das Bilderzeugungsmodell ein Maschinenlernmodell ist.

13. System nach Anspruch 12, wobei das eine oder die mehreren 2D-Erkundungsbilder wie folgt erhalten werden:
Überwachen der physiologischen Bewegung des Zielobjekts;
als Reaktion auf Erkennen, dass sich das Zielobjekt in einer mittleren und späten Diastole befindet, wenn die Herzbewegung des Zielobjekts gleichmäßig ist, Durchführen des Erkundungsscans, um das eine oder die mehreren 2D-Erkundungsbilder des Zielobjekts aufzunehmen.

14. System nach Anspruch 12 oder 13, wobei sich die innere Struktur des Zielobjekts aufgrund mindestens einem von einer Intervention einer Interventionsausrüstung in das Zielobjekt oder einer physiologischen Veränderung eines Interessenbereichs des Zielobjekts ändert.

15. Nichtflüchtiges, computerlesbares Medium, das mindestens einen Satz von Anweisungen umfasst, wobei der mindestens eine Satz von Anweisungen, wenn er von einem oder mehreren Prozessoren einer Rechenvorrichtung ausgeführt wird, verursacht, dass die Rechenvorrichtung ein Verfahren durchführt, wobei das Verfahren umfasst:
Erhalten (310) eines dreidimensionalen, 3D-, Basisbildes eines Zielobjekts, wobei das 3D-Basisbild durch Durchführen eines 3D-Scans an dem Zielobjekt in einer voreingestellten Haltung erfasst wird und das 3D-Basisbild eine Vielzahl von Einzelbildern beinhaltet, die einer Vielzahl von physiologischen Phasen des Zielobjekts entsprechen;
Erhalten (320) eines oder mehrerer zweidimensionaler, 2D-, Erkundungsbilder des Zielobjekts, wobei das eine oder die mehreren 2D-Erkundungsbilder des Zielobjekts durch Durchführen eines Erkundungsscans am Zielobjekt mit der voreingestellten Haltung aufgenommen werden, nachdem sich eine innere Struktur des Zielobjekts geändert hat;
Bestimmen einer physiologischen Zielphase des Zielobjekts entsprechend dem einen oder den mehreren 2D-Erkundungsbildern;
Auswählen eines Zieleinzelbildes aus der Vielzahl von Einzelbildern, das der physiologischen Zielphase entspricht;
Erzeugen (330) eines vorhergesagten 3D-Bildes des Objekts durch Verarbeiten des Zieleinzelbildes und des einen oder der mehreren 2D-Erkundungsbilder unter Verwendung eines Bilderzeugungsmodells, wobei das vorhergesagte 3D-Bild die innere Struktur des Zielobjekts anzeigt, wenn das eine oder die mehreren 2D-Erkundungsbilder aufgenommen werden, wobei das Bilderzeugungsmodell ein Maschinenlernmodell ist.

## Revendications

1. Procédé de génération d'image, mis en œuvre sur un dispositif informatique présentant un ou plusieurs processeurs et un ou plusieurs dispositifs de stockage, le procédé comprenant :
l'obtention (310) d'une image de base tridimensionnelle, 3D, d'un sujet cible, l'image de base 3D étant capturée par la réalisation d'un balayage 3D sur le sujet cible avec une posture prédéfinie, l'image de base 3D incluant une pluralité de trames correspondant à une pluralité de phases physiologiques du sujet cible ;
l'obtention (320) d'une ou plusieurs images de repérage bidimensionnelles, 2D, du sujet cible, la ou les images de repérage 2D du sujet cible étant capturées par la réalisation d'un balayage de repérage sur le sujet cible avec la posture prédéfinie après qu'une structure interne du sujet cible change ;
la détermination d'une phase physiologique cible du sujet cible correspondant à la ou aux images de repérage 2D ;
la sélection, parmi la pluralité de trames, d'une trame cible correspondant à la phase physiologique cible ;
la génération (330) d'une image 3D prédite du sujet par le traitement de la trame cible et de la ou des images de repérage 2D à l'aide d'un modèle de génération d'image, l'image 3D prédite indiquant la structure interne du sujet cible lorsque la ou les images de repérage 2D sont capturées, dans lequel le modèle de génération d'image est un modèle d'apprentissage automatique.

2. Procédé selon la revendication 1, dans lequel la ou les images de repérage 2D sont obtenues par :
la surveillance du mouvement physiologique du sujet cible ;
à la suite de la détection que le sujet cible est au milieu et à la fin d'une diastole lorsque le mouvement cardiaque du sujet cible est régulier, la réalisation du balayage de repérage pour capturer la ou les images de repérage 2D du sujet cible.

3. Procédé selon la revendication 1 ou 2, dans lequel la structure interne du sujet cible change en raison d'au moins un d'une intervention d'un équipement d'intervention dans le sujet cible et d'un changement physiologique d'une région d'intérêt du sujet cible.

4. Procédé selon la revendication 3, le procédé comprenant en outre :
l'obtention d'informations de référence se rapportant à au moins un d'un point d'intervention de l'équipement d'intervention, d'un itinéraire de déplacement de l'équipement d'intervention, d'une forme de l'équipement d'intervention, d'un matériau de l'équipement d'intervention et d'un type d'équipement d'intervention, dans lequel l'image 3D prédite est en outre générée sur la base des informations de référence.

5. Procédé selon la revendication 3 ou 4, dans lequel le modèle de génération d'image est formé à l'aide d'une pluralité d'échantillons d'entraînement, chacun d'eux incluant :
une image 3D vérité terrain et une ou plusieurs images de repérage 2D échantillons indiquant la structure interne d'un sujet échantillon avec un équipement d'intervention échantillon à l'intérieur du sujet échantillon, et
une image de base 3D échantillon indiquant la structure interne du sujet échantillon sans l'équipement d'intervention échantillon à l'intérieur du sujet échantillon, dans lequel le sujet cible est un patient qui doit être traité ou examiné, le sujet échantillon est un patient échantillon.

6. Procédé selon la revendication 5, dans lequel un échantillon d'entraînement de la pluralité d'échantillons d'entraînement est généré par :
la génération (410) de l'image de base 3D échantillon par la réalisation d'un premier balayage 3D sur le sujet échantillon avant que l'équipement d'intervention échantillon ne soit inséré dans le sujet échantillon ;
la génération (420) de l'image 3D vérité terrain par la réalisation d'un deuxième balayage 3D sur le sujet échantillon après que l'équipement d'intervention échantillon a été inséré dans le sujet échantillon ; et
la génération (430) de la ou des images de repérage 2D échantillons sur la base de l'image 3D vérité terrain.

7. Procédé selon la revendication 5, dans lequel un échantillon d'entraînement de la pluralité d'échantillons d'entraînement est généré par :
la génération (510) de l'image 3D vérité terrain par la réalisation d'un troisième balayage 3D sur le sujet échantillon après que l'équipement d'intervention échantillon a été inséré dans le sujet échantillon ; et
la génération (520) de l'image de base 3D échantillon et de la ou des images de repérage 2D échantillons sur la base de l'image 3D vérité terrain.

8. Procédé selon la revendication 7, dans lequel l'image de base 3D échantillon est générée sur la base de l'image 3D vérité terrain à l'aide d'un second modèle de génération d'image, le second modèle de génération d'image étant entraîné à l'aide d'une image d'entraînement du sujet échantillon capturée avec l'équipement d'intervention et d'une image d'entraînement des sujets échantillons capturée sans l'équipement d'intervention.

9. Procédé selon la revendication 5, dans lequel l'échantillon d'entraînement de la pluralité d'échantillons d'entraînement est généré par :
l'obtention (610) d'un modèle numérique représentant le sujet échantillon ;
la génération (620) de l'image de base 3D échantillon en simulant un balayage 3D sur le modèle numérique ; et
la génération (630) de l'image 3D vérité terrain et de la ou des images de repérage 2D échantillons sur la base de l'image de base 3D échantillon.

10. Procédé selon la revendication 1, dans lequel la génération de l'image 3D prédite par le traitement de la trame cible et de la ou des images de repérage 2D à l'aide du modèle de génération d'image comprend :
la génération d'une image 3D prédite préliminaire par le traitement de la trame cible et de la ou des images de repérage 2D à l'aide du modèle de génération d'image ; et
la génération de l'image 3D prédite par la réalisation d'une correction d'artefact sur l'image 3D prédite préliminaire à l'aide d'un modèle de correction d'artefact, le modèle de correction d'artefact étant entraîné conjointement avec le modèle de génération d'image.

11. Procédé selon l'une quelconque des revendications 1 à 10, dans lequel la ou les images de repérage incluent plusieurs images de repérage correspondant à différents angles de balayage.

12. Système de génération d'image, mis en œuvre sur un dispositif informatique présentant un ou plusieurs processeurs et un ou plusieurs dispositifs de stockage, le système comprenant :
au moins un dispositif de stockage incluant un ensemble d'instructions ; et
au moins un processeur configuré pour communiquer avec le au moins un dispositif de stockage, dans lequel, lors de l'exécution de l'ensemble d'instructions, le au moins un processeur est configuré pour ordonner au système de réaliser des opérations incluant :
l'obtention (310) d'une image de base tridimensionnelle, 3D, d'un sujet cible, l'image de base 3D étant capturée par la réalisation d'un balayage 3D sur le sujet cible avec une posture prédéfinie, l'image de base 3D incluant une pluralité de trames correspondant à une pluralité de phases physiologiques du sujet cible ;
l'obtention (320) d'une ou plusieurs images de repérage bidimensionnelles, 2D, du sujet cible, la ou les images de repérage 2D du sujet cible étant capturées par la réalisation d'un balayage de repérage sur le sujet cible avec la posture prédéfinie après qu'une structure interne du sujet cible change ;
la détermination d'une phase physiologique cible du sujet cible correspondant à la ou aux images de repérage 2D ;
la sélection, parmi la pluralité de trames, d'une trame cible correspondant à la phase physiologique cible ;
la génération (330) d'une image 3D prédite du sujet par le traitement de l'image de trame cible et de la ou des images de repérage 2D à l'aide d'un modèle de génération d'image, l'image 3D prédite indiquant la structure interne du sujet cible lorsque la ou les images de repérage 2D sont capturées, dans lequel le modèle de génération d'image est un modèle d'apprentissage automatique.

13. Système selon la revendication 12, dans lequel la ou les images de repérage 2D sont obtenues par :
la surveillance du mouvement physiologique du sujet cible ;
à la suite de la détection que le sujet cible est au milieu et à la fin d'une diastole lorsque le mouvement cardiaque du sujet cible est régulier, la réalisation du balayage de repérage pour capturer la ou les images de repérage 2D du sujet cible.

14. Système selon la revendication 12 ou 13, dans lequel la structure interne du sujet cible change en raison d'au moins un d'une intervention d'un équipement d'intervention dans le sujet cible et d'un changement physiologique d'une région d'intérêt du sujet cible.

15. Support non transitoire lisible par ordinateur comprenant au moins un ensemble d'instructions, dans lequel, lorsqu'il est exécuté par un ou plusieurs processeurs d'un dispositif informatique, le au moins un ensemble d'instructions amène le dispositif informatique à réaliser un procédé, le procédé comprenant :
l'obtention (310) d'une image de base tridimensionnelle, 3D, d'un sujet cible, l'image de base 3D étant capturée par la réalisation d'un balayage 3D sur le sujet cible avec une posture prédéfinie, l'image de base 3D incluant une pluralité de trames correspondant à une pluralité de phases physiologiques du sujet cible ;
l'obtention (320) d'une ou plusieurs images de repérage bidimensionnelles, 2D, du sujet cible, la ou les images de repérage 2D du sujet cible étant capturées par la réalisation d'un balayage de repérage sur le sujet cible avec la posture prédéfinie après qu'une structure interne du sujet cible change ;
la détermination d'une phase physiologique cible du sujet cible correspondant à la ou aux images de repérage 2D ;
la sélection, parmi la pluralité de trames, d'une trame cible correspondant à la phase physiologique cible ;
la génération (330) d'une image 3D prédite du sujet par le traitement de la trame cible et de la ou des images de repérage 2D à l'aide d'un modèle de génération d'image, l'image 3D prédite indiquant la structure interne du sujet cible lorsque la ou les images de repérage 2D sont capturées, dans lequel le modèle de génération d'image est un modèle d'apprentissage automatique.
